# EUROPEAN PATENT APPLICATION

(11) **EP 4 155 101 A1**
(43) Date of publication of application: **29.03.2023**
(21) Application number: 20936505.5
(22) Date of filing: 20.05.2020
(51) Int. Cl.: B60H 3/06, A61L 9/20, B01D 53/86, B65F 3/00

(54) **DEVICE FOR AIR PURIFICATION, INCORPORATED INTO A VEHICLE**

(71) Applicant: AGM Human Consulting, S.L., 28232 Las Rozas-Madrid (ES)
(72) Inventor: MORO FRANCO, Eusebio, 28232 LAS ROZAS-MADRID (ES)
(74) Representative: Urizar Anasagasti, Jesus Maria
(86) International application number: PCT/ES2020/070326
(87) International publication number: WO 2021/234186

(57) **Abstract**

Disclosed is a device for air purification, incorporated into a vehicle, which comprises a case (1), fixed to the outside of the vehicle (13) and having a longitudinal duct (12) through which air passes, the air entering impure through at least one opening (2) located in the front part, with respect to the direction of travel of the vehicle, and exiting through at least one opening (3) located in the rear part under the suctioning effect of at least one fan (10) disposed in the opening (3). From the inlet (2) to the outlet (3), the air purification device successively includes the following devices: a photocatalytic reactor; a reverse microwave (6); an activated carbon filter (8); and a HEPA filter, just before the fans (10) located in the outlet conduit (3).

## Description

### Technical field

The sector in which the invention is comprised is that of air purification, more specifically that of the manufacture of devices to reduce the concentration of particles suspended in the air, capable of producing diseases (viruses, bacteria, etc.), odors that can be an environmental and health problem, such as those generated in industrial activities, soot, tobacco smoke, etc., and other drawbacks, such as those caused by dust or pollen, especially when they occur in places close to residential areas.

The present invention consists of a device for air purification which has the special feature of being incorporated into any vehicle, whether air, space or land vehicles. Basically, this device comprises a support structure that incorporates filtering units in its interior intercalated between an impure air inlet and an outlet for the now clean air, the impurities of which have been neutralized and retained by the aforementioned filtering units. It is intended for purifying the outdoor environment, especially the urban environment, such as parks, roads, traffic circles, etc. The vehicle carrying this device, whether or not the drive thereof has polluting characteristics, becomes an element that supplies to the environment cleaner air than what it takes in, the dirt particles remaining in the device itself, thus defining an important means to counteract the pollution of the air to which the device is applied.

### State of the art

The social demand for cleaner and odor-free air has led to the development of increasingly restrictive regulations regarding the emission of harmful and unpleasant gases into the atmosphere, which has led to the emergence of increasingly more filtration devices, some of which are fitted in automotive vehicles to capture and filter the air, both for inside the vehicle and for expulsion into the atmosphere, cleaning the air of fumes, suspended particles, pollen and other pollutants.

However, converting a vehicle into a mobile air purifier has not become widespread, although there are precedents in this regard, such as document WO2012152967, by the same inventor, which describes a device with air ducts in which a fan-aspirator is intercalated. Passive and mechanical air-filtration elements are placed before the formed by the ducts and the fan-aspirator. At the outlet of the fan-aspirator there is an active filtration module in which one or more non-thermal plasma ducts are included, so that when the ducts closest to said module bifurcate or branch inside said module, they define at least two clean air outlets. The components of the assembly are connected to a control unit, connected to the vehicle and the control unit by means of a communication that can be mechanical, electrical, electronic, optoelectronic or any combination thereof. Document WO2015086871, by the same inventor, incorporates a catalytic module, an ionizing module and a photocatalytic module connected to the main duct between the inlet conduits and the fan.

Document CN109927514 describes an intelligent solar air purifier, which includes a casing open at both open ends in which a sponge is installed, the sponge being impregnated with a liquid that acts as a filter-humectant, then an activated carbon adsorption filter and a HEPA filter, and finally a titanium dioxide photocatalyst step and an ultraviolet light sterilization step, all inside the casing in order from the body of the sponge to a suction fan located at the outlet. The upper part includes photovoltaic panels that generate the energy necessary for the operation of all these devices.

However, no device for purifying air presenting the architecture, application possibilities and feasibility described in the present invention is known in the current state of the art. This device significantly improves any of the documents mentioned above by including a reverse microwave, as can be seen in the practical example of application and by means of the set of claims filed.

### Description of the invention

Based on the prior art, an objective of the present invention is to provide a device for air purification, capable of being incorporated into any vehicle, which solves the problem posed of purifying a large air flow (approximately 4000 m³/hour), thereby reducing pollution and improving ambient air quality by simply installing such devices, for example, in buses circulating in a large city.

In order to achieve the proposed objectives mentioned in the previous section, the invention proposes a device for air purification, incorporated into a vehicle, having the features of claim 1.

In this device, the polluted air is absorbed by the front part through a nozzle that distributes the air flow through a network of filtering elements. Finally, the air passes through the various elements of the device which are responsible for purifying the impure air, retaining the pollutants and expelling the treated air through a nozzle located in the rear part of the device. Both the inlet and outlet conduits are connected by means of a main duct, where the filtering elements are incorporated. At the outlet opening of said duct there is at least one fan that generates a depression helping the air circulation from the inlet conduit to the outlet conduit through said filtering elements.

The device first has a reactor in which a heterogeneous photocatalysis process takes place to remove volatile organic compounds, this process being based on the oxidation of these undesirable compounds present in the air by means of a semiconductor catalyst that is activated by light of a certain wavelength. Therefore, the components necessary for the photocatalysis reaction to take place are a compound to be degraded, an oxidizing compound such as the oxygen contained in the air, a medium where the reaction takes place, in this case the air itself, a photocatalyst such as titanium dioxide or another semiconductor compound, and a source of ultraviolet light, either natural (from the sun) or artificial (lamps). The invention proposes a specific photocatalytic reactor, in which titanium dioxide is used to impregnate steel plates, which are located in an intercalated manner, with some in the lower part and the other two being in the upper part, with this arrangement reducing the air speed, while at the same time facilitating that the impure air entering through the front part hits against these plates to make a previous decantation so that the rest of the filtering elements are more protected in a first phase. Downstream of these plates and towards the exit, ultraviolet light tubes are placed sequentially to achieve a good photocatalytic reaction.

One of the improvements incorporated in this device, which makes it much more efficient than those existing in the state of the art, is a reverse microwave system, intercalated in said duct before the filtering elements, the function of which is to substantially reduce the temperature of the air stream to achieve a better capture of suspended particles and pollutants, since in this way they do not sublimate and their volume is reduced, thus achieving a much more efficient capture, destroying the molecular part of the different pollutants. Otherwise, the incoming impure air stream would be more dissipated, making it more difficult to capture pollutants inside the compartment.

Subsequently, an activated carbon filter is arranged for the purpose of absorbing harmful particles and gases, being connected to a differential pressure clock located in the cabin of the vehicle, which will indicate the state of the filter, since if it is saturated with pollutants, the loss of pressure increases, along with its saturation. In this case it will be necessary to remove this element and regenerate it by means of a sonic system before reinstalling it again.

Finally, a HEPA filter is arranged, just before the fans, which filters pollutants that may have passed through the activated carbon filter and have not been purified.

All these elements are located inside an aerodynamic compartment that is placed in the upper part of the truck or bus and will be arranged horizontally, designed from less to more so that it does not have a braking effect with the vehicle, allowing the vehicle to be, as well as a means of transportation, an effective means of combating pollution.

This device will be in operation when the vehicle is in motion or stopped at a traffic light. The power supply for the electrical and electronic components, such as fans, lamps, sensors, etc., can be supplied from the vehicle's system or, preferably, the device will be autonomous, for which purpose a photovoltaic panel is installed in the upper part of the device.

A massive implementation of this device in cities with health problems due to atmospheric pollution mostly caused by road traffic would considerably improve air quality and reduce the concentration of particles suspended in the air, capable of producing diseases (viruses, bacteria, etc.), odors that can be an environmental and health problem.

This type of device can also be coupled to purify the air inside a vehicle, bus, railroad carriage, etc. In this case, the air that is purified is the air inside the vehicle, which facilitates a more shared use in cases of epidemics, given that since viruses and bacteria are eliminated from the environment, the safety distance between travelers can be reduced.

The system does not require any additional product or expense, as the TiO₂-based technology is virtually self-sufficient and inexhaustible. The device can be installed in new or old vehicles.

Another purpose of the invention consists of its application to any conventional vehicle without significant incompatibilities with respect to makes, types of ventilation used and other characteristics.

### Description of the drawings

As a complement to the description being made, and for the purpose of helping to make the features of the invention more readily understandable, the present specification is accompanied by a set of drawings which, by way of illustration and not limitation, represent the following:
Figure 1 shows a schematic vertical-longitudinal section view of a device made according to the invention.
Figure 2 shows a rear elevation view from the purified air outlet of this device.
Figure 3 schematically depicts the assembly of the filters (8, 9) in the casing (1).
Figure 4 schematically depicts the assembly of the device for purifying the air inside a bus (13).

The following is a list of all the references included in the attached drawings.
1) Casing.
2) Impure air inlet.
3) Clean air outlet.
4) Units of sheet metal impregnated with titanium dioxide (TiO₂).
5) Units of ultraviolet light tube.
6) Reverse microwave.
7) Adjustable grid.
8) Activated carbon filter.
9) HEPA filter.
10) Fans.
11) Inlet grid.
12) Inner duct.
13) Support vehicle.
14) Support guides for the filters (8, 9).
15) Air intake tube in the vehicle.
16) Air inlet tube in the vehicle.

### Embodiment of the invention

As can be observed in the mentioned figures, the device for air purification, incorporated into a vehicle, object of the invention, comprises a casing (1), which is fixed on the outside of the vehicle (13), preferably in the upper part thereof if it is a land vehicle. This casing (1) has a longitudinal duct (12) through which air passes, the air entering impure through at least one opening (2) located in the front part, with respect to the direction of travel of the vehicle, and exiting through at least one opening (3) located in the rear part under the suction effect of at least one fan (10) disposed in the opening (3). Between the inlet (2) and outlet (3), the device successively includes, in this order, the following devices: A photocatalytic reactor, a reverse microwave (6), an activated carbon filter (8) and a HEPA filter (9).

The photocatalytic reactor consists of several steel plates (4), which are impregnated with titanium dioxide, and of ultraviolet light tubes (5), sequentially located to perform a good photocatalytic reaction, located downstream of said plates (4) in the clean air outlet direction. On the other hand, the mentioned plates (4) are located in an intercalated manner, with some in the lower part and others in the upper part of said duct, forming a labyrinth that reduces air speed, while at the same time facilitating that the impure air entering through the opening (2) hits against these plates (4) so that a previous decantation of various suspended particles takes place, so that the rest of the filtering elements are protected in this first phase. The photons, together with TiO2, destroy the pollutant that may be in the air stream being sucked in from the outside. These tubes should be replaced approximately every twelve months to ensure that the light is intense enough to degrade pollutants.

An inverted microwave (6) is installed downstream of the photocatalytic reactor. The purpose of this device is to reduce the temperature of the inlet air stream in order to better capture the pollutant particles by not sublimating them and reducing their volume, making their capture more efficient; it also generates a magnetic field that abates the pollutant. This element does not need to be replaced, just like the plates (4) placed first.

Next, two filters are placed, a first activated carbon filter (8), for the purpose of absorbing harmful particles and gases; and a second HEPA filter, just before the fan(s) (10) located in the outlet conduit (3), said filtering being for the purpose of filtering the pollutants that may have passed through the activated carbon filter and have not been purified.

The activated carbon filter (8) is connected to a differential pressure clock, which is located in a visible place in the cabin of the vehicle; this clock indicates the state of the filter, that is, if it is saturated with pollutant, since in this case the loss of pressure increases, along with the saturation of the filter, and therefore this would indicate that its replacement or regeneration by means of a sonic system is necessary.

The casing (1) that houses all the devices forms an aerodynamic compartment that is placed horizontally in the upper part of the truck or bus and has side accesses, at least in correspondence with the filters (8, 9), that allow their removal for cleaning or replacement, without the need to disassemble the device in its entirety, being moved to one side by transverse guides (14).

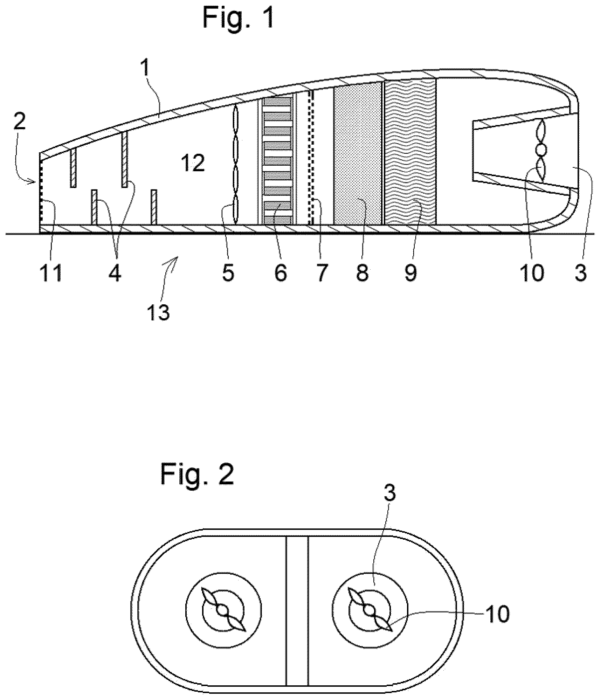

In Figure 1, an adjustable grid (7) can be seen between the reverse microwave (6) and the activated carbon filter (8).

Likewise, a grid (11) is placed at the inlet opening (2) that prevents the entry of insects, leaves or large particles inside the device due to the absorption of the air by the fans (10) from the outlet opening.

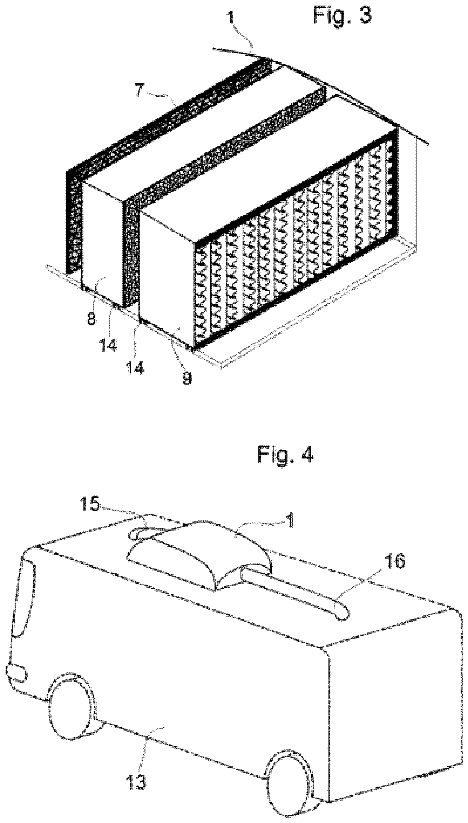

When it comes to purifying the air inside the vehicle (13) in which the device (1) is assembled, the inlet opening (2) and outlet opening (3) of the device are connected by means of separate tubes (15, 16) with the inside of the compartment of the vehicle (13) for the purpose of purifying the air inside the vehicle. In the example shown in Figure 4, the device is assembled on the roof of a bus and the air intake into the device is taken from inside the vehicle, preferably from the front area, while the purified air outlet is also directed to the inside of said vehicle, preferably in the rear area. The position of the air inlet and outlet inside the bus is not a relevant fact, the only feature being that they will be separated as far as the interior space allows in order to allow the purified air to travel as far as possible inside the vehicle.

The ultraviolet light tubes (5), fans (10) and other electrical/electronic devices are powered from the vehicle current or from a photovoltaic plate fixed on top of the device.

A device with an approximate electrical power of 1500 W is expected to provide a flow rate of 4000 m3/hour of clean air, with a purification level of more than 99%.

The pollutants captured are: CO₂, CO, NOₓ, SO₂, benzene, formaldehyde, pathogens and suspended particles (PM10 and PM2.5).

Having sufficiently described the nature of the invention, as well as a preferred exemplary embodiment thereof, it is evident that the invention can have industrial applicability in the indicated sector.

Likewise, it is stated for the appropriate purposes that the materials, shape, size and arrangement of the elements described may be modified, as long as this does not imply an alteration of the essential features of the invention that are claimed below:

## Claims

1. A device for air purification, incorporated into a vehicle, **comprising** a casing (1), fixed to the outside of the vehicle (13), preferably in the upper part of the vehicle if it is a land vehicle, and having a longitudinal duct (12) through which air passes, the air entering impure through at least one opening (2) located in the front part, with respect to the direction of travel of the vehicle, and exiting through at least one opening (3) located in the rear part under the suction effect of at least one fan (10) disposed in the opening (3); from the inlet (2) to the outlet (3), the air purification device successively includes the following devices:
- a photocatalytic reactor consisting of several steel plates (4), which are impregnated with titanium dioxide, and of ultraviolet light tubes (5), sequentially located to perform a good photocatalytic reaction and downstream of said plates (4) in the clean air outlet direction;
- a reverse microwave (6) for the purpose of reducing the temperature of the inlet air stream in order to better capture the pollutant particles by not sublimating them and reducing their volume, making their capture more efficient; it also generates a magnetic field that abates the pollutant;
- an activated carbon filter (8), for the purpose of absorbing harmful particles and gases; and
- a HEPA filter, just before the fans (10) located in the outlet conduit (3), for the purpose of filtering the pollutants that may have passed through the activated carbon filter and have not been purified.

2. The device according to claim 1, **characterized in that** the plates (4), which are impregnated with titanium dioxide, are located in an intercalated manner, with some in the lower part and others in the upper part of said duct, forming a labyrinth that reduces air speed, while at the same time facilitating that the impure air entering through the opening (2) hits against these plates (4) so that a previous decantation of various suspended particles takes place, in order to protect the rest of the existing filtering elements up to the outlet opening (3).

3. The device according to any of the preceding claims, **characterized in that** the casing (1) that houses all the devices forms an aerodynamic casing that is placed horizontally in the upper part of the truck or bus and has side accesses, at least in correspondence with the filters (8, 9), that allow their removal for cleaning or replacement, without the need to disassemble the device in its entirety.

4. The device according to any of the preceding claims, **characterized in that** the activated carbon filter (8) is connected to a differential pressure clock that indicates its clogging state.

5. The device according to any of the preceding claims, **characterized in that** an adjustable grid (7) is placed between the reverse microwave (6) and the activated carbon filter (8).

6. The device according to any of the preceding claims, **characterized in that** the ultraviolet light tubes (5), fans (10) and other electrical / electronic devices are powered from the vehicle current or from at least one photovoltaic plate fixed on top of the device.

7. The device according to any of the preceding claims, **characterized in that** there is a grid (11) at the inlet opening (2) that prevents the entry of insects, leaves or large particles inside the device due to the absorption of the air by the fans (10) from the outlet opening.

8. The device according to any of the preceding claims, **characterized in that** the inlet opening (2) and outlet opening (3) are connected by means of tubes (15, 16) to the inside of the compartment of the vehicle (13) in which they are assembled in order to purify the air existing inside the vehicle.
